# FASCICULE DE BREVET EUROPEEN

(11) **EP 2 768 433 B1**
(45) Date de publication et mention de la délivrance du brevet: **10.08.2016**
(21) Numéro de dépôt: 12787760.3
(22) Date de dépôt: 05.10.2012
(51) Int. Cl.: A61F 2/44

(54) **PERFECTIONNEMENTS AUX IMPLANTS NUCLÉIQUES EN SILICONE**
VERBESSERUNGEN AN NUKLEAREN SILIZIUMIMPLANTATEN
IMPROVEMENTS TO SILICON NUCLEAR IMPLANTS

(30) Priorité: 17.10.2011 FR 1159341
(43) Date de publication de la demande: 27.08.2014
(73) Titulaire: Clariance, 62000 Dainville (FR)
(72) Inventeur: TORNIER, Alain, F-38330 Saint Ismier (FR); VIART, Guy, F-62128 Saint Leger (FR); LEROY, Jean Yves, F-62870 Campagne-les-Hesdin (FR); BILLON, Adrien, F-59790 Rochin (FR)
(74) Mandataire: Jeannet, Olivier
(86) Numéro de dépôt international: PCT/FR2012/052258
(87) Numéro de publication internationale: WO 2013/057409

(56) Documents cités:
- WO-A1-2010/077359
- WO-A2-02/17825
- WO-A2-2006/020531
- US-A1- 2008 065 076
- US-A1- 2009 143 716

## Description

La présente invention est relative à un implant nucléique en silicone constituant entre deux vertèbres sus et sous jacentes d'un segment rachidien un dispositif d'étaiement intervertébral amortissant et assurant la mobilité de l'unité fonctionnelle formée par lesdites vertèbres.

La dégénérescence du nucléus est en général le premier facteur qui conduit à la dégradation du disque et conséquemment des facettes articulaires. Cette dégénérescence peut provoquer des hernies et la technique de nucléotomie est souvent choisie pour traiter ce problème.

Toutefois, même si la douleur disparaît en post op immédiat, la perte de hauteur du disque et l'hyper-mobilité du segment consécutive accentuent progressivement la dégradation du segment rachidien avec des douleurs qui réapparaissent et parfois avec la dégradation des étages adjacents.

Le remplacement du nucléus de manière précoce pour de tels patients, peut représenter une alternative efficace au remplacement total du disque ou à la fusion du segment.

En effet, un maintien d'une hauteur intervertébrale suffisante avec une mobilité quasi normale peut apporter une certaine stabilité du segment et ainsi ralentir voire stopper les phénomènes de dégénérescence.

On connait d'après la demande de brevet international WO 2009/130417 appartenant au demandeur un implant nucléique comportant au moins un élément de remplissage constitué d'au moins un fil continu agencé, à l'intérieur d'un espace nucléique obtenu après nucléotomie du disque intervertébral, suivant un profil en forme de couronne dont l'empilement des spires permet de délimiter un espace interne central qui est rempli d'un produit qui peut être un gel ou un produit pâteux ou un produit à base de fibres ou un matériau viscoélastique injectable.

On note que ce type d'implant présente des problèmes de migration progressive de ce dernier vers le canal médullaire lors de mouvements répétés de flexion extension desdites vertèbres du segment rachidien car l'implant n'est pas relié ni attaché au corps de la vertèbre correspondante.

L'implant nucléique suivant la présente invention a pour objet de perfectionner l'implant nucléique en réduisant le nombre de ses composants et en garantissant une liaison stable et efficace entre l'implant et les corps vertébraux du segment rachidien concerné.
L'implant nucléique suivant la présente invention est constitué d'un noyau agencé à l'intérieur d'un espace nucléique obtenue après nucléotomie du disque intervertébral et d'au moins une prolongation pénétrant à l'intérieur d'au moins un canal ménagé dans le corps vertébral de la vertèbre correspondante pour renforcer et assurer entre l'implant nucléique et le corps osseux de la vertèbre une liaison par diffusion ou migration dans l'os spongieux de ladite vertèbre de la matière viscoélastique constituant ledit implant nucléique.

L'implant nucléique suivant la présente invention comporte un noyau comprenant au moins deux prolongations qui s'étendent de part et d'autre du centre dudit noyau.

L'implant nucléique suivant la présente invention comporte un noyau et des prolongations qui sont obtenus à partir d'une composition fluide ou de type pâte à base d'organosiloxane, autopolymérisant à température ambiante en silicone à élasticité permanente.

L'implant nucléique suivant la présente invention comporte un noyau et des prolongations qui sont obtenus à partir d'une composition à base de polydiméthylsiloxane.

L'implant nucléique suivant la présente invention comporte un noyau et des prolongations qui sont obtenus à partir d'une composition présentant une teneur en catalyseur au platine.

L'implant nucléique suivant la présente invention comporte un noyau et des prolongations qui sont obtenus à partir d'une composition contenant un catalyseur à base de siloxane à fonctionnalité hydrure et de siloxane à fonctionnalité vinyle.

La description qui va suivre en regard des dessins annexés, donnés à titre d'exemples non limitatifs permettra de mieux comprendre l'invention, les caractéristiques qu'elle présente, et les avantages qu'elle est susceptible de procurer :
Figure 1 est une vue en perspective éclatée illustrant l'implant nucléique suivant la présente invention dont le noyau est obtenue par l'injection d'une matière à base de silicone.
Figure 2 est une vue en coupe illustrant l'implant nucléique suivant la présente invention dont le noyau est obtenue par l'injection d'une matière à base de silicone.

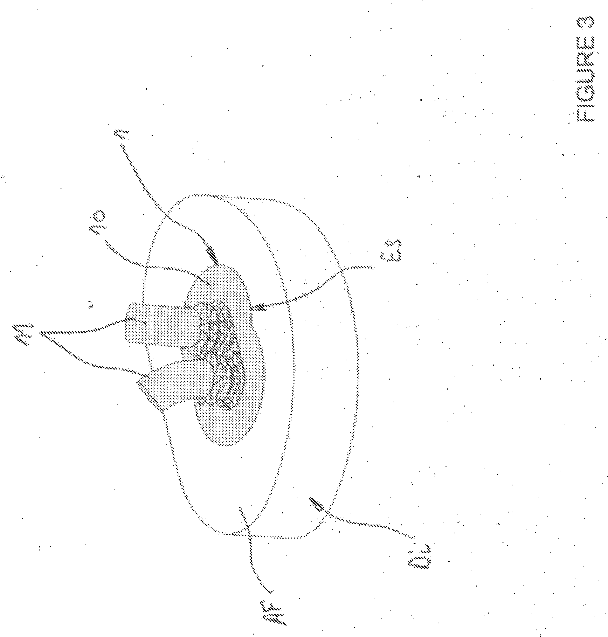

Figure 3 est une vue en perspective représentant schématiquement l'implant nucléique dont le noyau comporte par exemple au moins deux cordons s'étend de part et d'autre du centre dudit noyau suivant la présente invention.

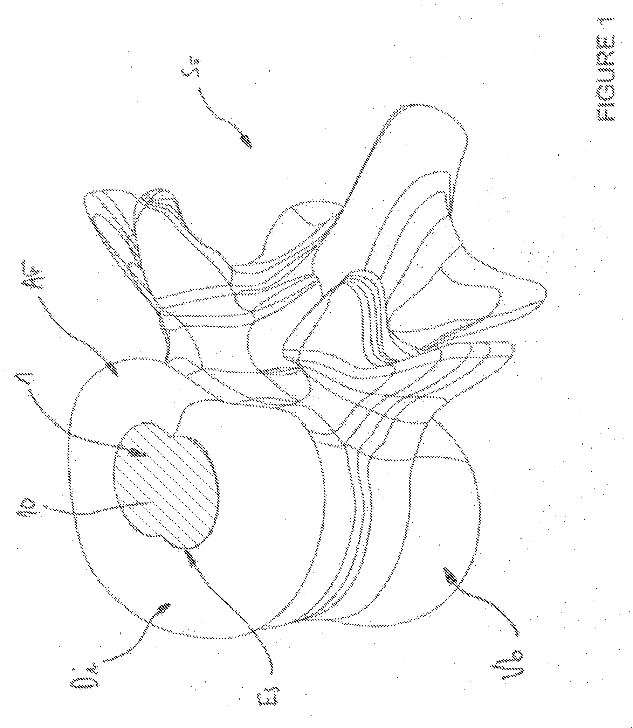

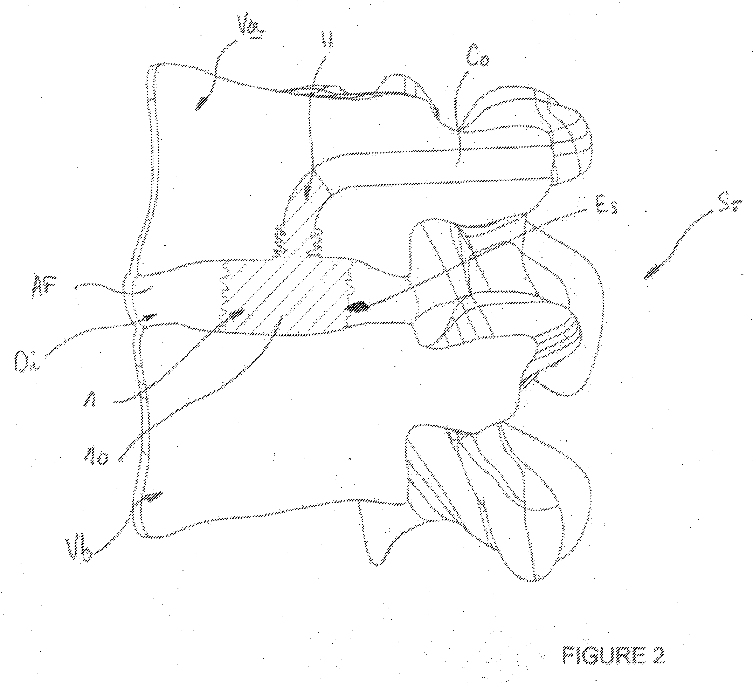

On a représenté en figures 1 et 2 un segment rachidien Sr d'une colonne vertébrale dont l'une au moins des vertèbres sus jacente Va et sous jacente Vb va être percée par l'intermédiaire d'un dispositif de forage, non représenté, d'au moins un canal osseux à profil courbe Co afin d'atteindre la face supérieure d'un disque intervertébral Di endommagé.

Le forage à profil courbe, par exemple, de la vertèbre sus jacente Va permet de réaliser un abord chirurgical percutané trans-osseux pour atteindre le nucleus du disque intervertébral Di de manière à pouvoir par la suite effectuer les interventions nécessaires sur le disque intervertébral endommagé jusqu'à obtenir au centre de « l'annulus fibrosus » AF un espace nucléique Es.

Le forage à profil courbe des vertèbres sus jacente Va et/ou sous jacente Vb est réalisé, par exemple, au moyen d'un dispositif de forage décrit et protégé dans la demande de brevet FR11/00199 appartenant au demandeur.

Lorsque que le canal osseux à profil courbe Co est obtenu, ce dernier permet au moyen d'un dispositif d'injection, non représenté, l'introduction d'une matière viscoélastique auto polymérisant à température ambiante permettant la réalisation à l'intérieur de l'espace nucléique Es d'un implant nucléique 1.

L'implant nucléique 1 est réalisé en silicone ou dans une matière viscoélastique auto polymérisant à température ambiante. L'implant nucléique 1 est constitué d'un noyau 10 comportant au moins une prolongation 11 pénétrant à l'intérieur du ou des canaux à profil courbe Co ménagé dans le corps vertébral de la vertèbre correspondante.

Chaque prolongation 11 s'étendant à partir du noyau 10 permet d'assurer une liaison entre ledit noyau et le corps osseux de la vertèbre Va et/ou Vb correspondante évitant toute possibilité de migration de l'implant nucléique 1 par rapport à « l'annulus fibrosus » AF et auxdites vertèbres.

Chaque prolongation 11 permet de renforcer et d'assurer entre l'implant nucléique 1 et le corps osseux de la vertèbre Va et/ou Vb une liaison par diffusion ou migration de la matière viscoélastique auto polymérisant à température ambiante dans l'os spongieux dudit corps vertébral de la vertèbre Va, Vb correspondante.

En effet le lien réalisé par les prolongations 11 permet de maintenir l'implant nucléique 1 en place entre les vertèbres Va et/ou Vb et évite ainsi la migration progressive de ce dernier vers le canal médullaire lors de mouvements répétés de flexion extension desdites vertèbres Va et/ou Vb du segment rachidien Sr.

Egalement chaque prolongation 11 de l'implant nucléique 1 permet d'obstruer le canal osseux courbe Co correspondant pour empêcher toute possibilité de migration de corps étranger à l'intérieur du disque intervertébral Di.

L'implant nucléique 1 formé du noyau 10 et des prolongations 11 peuvent être obtenus à partir d'une composition fluide ou de type pâte à base d'organosiloxane, auto polymérisant à température ambiante en silicone à élasticité permanente.

L'implant nucléique 1 formé du noyau 10 et des prolongations 11 sont obtenus à partir d'une composition fluide ou de type pâte à base d'organosiloxane de type polydiméthylsiloxane auto polymérisant à température ambiante en silicone à élasticité permanente.

L'implant nucléique 1 formé du noyau 10 et des prolongations 11 sont obtenus à partir d'une composition fluide ou de type pâte à base d'organosiloxane pouvant être de type polydiméthylsiloxane auto polymérisant à température ambiante en silicone à élasticité permanente et présentant une teneur en catalyseur au platine.

L'implant nucléique 1 formé du noyau 10 et des prolongations 11 sont obtenus à partir d'une composition fluide ou de type pâte à base d'organosiloxane pouvant être de type polydiméthylsiloxane auto polymérisant à température ambiante en silicone à élasticité permanente contenant un catalyseur à base de siloxane à fonctionnalité hydrure et de siloxane à fonctionnalité vinyle.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Implant nucléique constituant entre deux vertèbres sus jacente (Va) et sous jacentes (Vb) d'un segment rachidien (Sr) un dispositif d'étaiement intervertébral amortissant et assurant la mobilité de l'unité fonctionnelle formée par lesdites vertèbres, **caractérisé en ce qu'**il est constitué d'un noyau (10) agencé à l'intérieur d'un espace nucléique (Es) obtenue après nucléotomie du disque intervertébral (Di) et d'au moins une prolongation (11) pénétrant à l'intérieur d'au moins un canal (Co) ménagé dans le corps vertébral de la vertèbre (Va, Vb) correspondante pour renforcer et assurer entre l'implant nucléique (1) et le corps osseux de la vertèbre (Va) et/ou (Vb) une liaison par diffusion ou migration dans l'os spongieux de ladite vertèbre de la matière viscoélastique constituant ledit implant nucléique.

2. Implant nucléique suivant la revendication 1, **caractérisé en ce que** le noyau (10) comporte au moins deux prolongations (11) s'étendant de part et d'autre du centre dudit noyau.

3. Implant nucléique suivant la revendication 2, **caractérisé en ce que** le noyau (10) et les prolongations (11) dudit implant sont obtenus à partir d'une composition fluide ou de type pâte à base d'organosiloxane, autopolymérisant à température ambiante en silicone à élasticité permanente.

4. Implant nucléique suivant la revendication 3, **caractérisé en ce que** le noyau (10) et les prolongations (11) dudit implant sont obtenus à partir d'une composition à base de polydiméthylsiloxane.

5. Implant nucléique suivant l'une des revendications 3 et 4, **caractérisé en ce que** le noyau (10) et les prolongations (11) dudit implant sont obtenus à partir d'une composition présentant une teneur en catalyseur au platine.

6. Implant nucléique suivant l'une des revendications 2 à 3, **caractérisé en ce que** le noyau (10) et les prolongations (11) dudit implant sont obtenus à partir d'une composition contenant un catalyseur à base de siloxane à fonctionnalité hydrure et de siloxane à fonctionnalité vinyle.

## Patentansprüche

1. Nukleares Implantat, das zwischen zwei darüberliegenden (Va) und darunterliegenden (Vb) Wirbeln eines Wirbelsegments (Sr) eine stoßdämpfende Zwischenwirbelstützvorrichtung bildet, die die Beweglichkeit der durch die Wirbel gebildeten Funktionseinheit sicherstellt, **dadurch gekennzeichnet, dass** es aus einem Kern (10), der im Inneren eines nuklearen Raumes (Es) angeordnet ist, der nach der Nukleotomie der Bandscheibe (Di) erhalten wird, und aus mindestens einer Verlängerung (11), die das Innere von mindestens einem im Wirbelkörper des entsprechenden Wirbels (Va, Vb) ausgebildeten Kanal (Co) penetriert, besteht, um zwischen dem nuklearen Implantat (1) und dem Knochenkörper des Wirbels (Va) und/oder (Vb) eine Verbindung zur Diffusion oder Migration im spongiösen Knochen des Wirbels aus dem viskoelastischen Stoff, der das nukleare Implantat bildet, zu verstärken und sicherzustellen.

2. Nukleares Implantat nach Anspruch 1, **dadurch gekennzeichnet, dass** der Kern (10) mindestens zwei Verlängerungen (11) aufweist, die sich beiderseits der Mitte des Kerns erstrecken.

3. Nukleares Implantat nach Anspruch 2, **dadurch gekennzeichnet, dass** der Kern (10) und die Verlängerungen (11) des Implantats aus einer fluiden oder pastenartigen Zusammensetzung auf der Grundlage von Organosiloxan, das bei Umgebungstemperatur zu dauerelastischem Silizium autopolymerisiert, erhalten werden.

4. Nukleares Implantat nach Anspruch 3, **dadurch gekennzeichnet, dass** der Kern (10) und die Verlängerungen (11) des Implantats aus einer Zusammensetzung auf Grundlage von Polydimethylsiloxan erhalten werden.

5. Nukleares Implantat nach einem der Ansprüche 3 und 4, **dadurch gekennzeichnet, dass** der Kern (10) und die Verlängerungen (11) des Implantats aus einer Zusammensetzung erhalten werden, die einen Gehalt eines Platinkatalysators aufweist.

6. Nukleares Implantat nach einem der Ansprüche 2 bis 3, **dadurch gekennzeichnet, dass** der Kern (10) und die Verlängerungen (11) des Implantats aus einer Zusammensetzung erhalten werden, die einen Katalysator auf Grundlage von Siloxan mit Hydridfunktionalität und Siloxan mit Vinylfunktionalität enthält.

## Claims

1. A "spinal nuclear implant" comprised of an inter-vertebral device disposed between two vertebrae, namely between an overlying vertebra (Va) and an underlying vertebra (Vb), of a given spinal segment (Sr), which device serves to support, provide damping, and ensureg the mobility of the functional unit formed by the said vertebrae, **characterised in that** device is comprised of:
-- a core (10) disposed inside of a nuclear space (Es) obtained after nucleotomy of an intervertebral disc (Di), and
-- at least one extension (11) which penetrates into the interior of at least one channel (Co) formed in the vertebral body of the corresponding vertebra (Va, Vb),
in order to strengthen, and in order to provide a link, between the "nuclear implant" (1) and the osseous body of the vertebra (Va and/or Vb), by means of diffusion or migration in the cancellous bone of said vertebra of the viscoelastic material comprising said "nuclear implant".

2. The "spinal nuclear implant" according to claim 1, **characterised in that** the core (10) is comprised of at least two extensions (11) which extend on respective sides of the centre of said core.

3. The "spinal nuclear implant" according to claim 2, **characterised in that** the core (10) and the extensions (11) of said implant are obtained from a fluid or pasteous composition based on organosiloxane which is autopolymerised at ambient temperature to form a silicone with a permanent elasticity.

4. The "spinal nuclear implant" according to claim 3, **characterised in that** the core (10) and the extensions (11) of said implant are obtained from a composition based on polydimethylsiloxane.

5. The "spinal nuclear implant" according to one of claims 3-4, **characterised in that** the core (10) and the extensions (11) of said implant are obtained from a composition which has a content of a platinum catalyst.

6. The "spinal nuclear implant" according to one of claims 2-3, **characterised in that** the core (10) and the extensions (11) of said implant are obtained from a composition which has a content of a catalyst based on a siloxane which has a hydride functional group and a siloxane which has a vinyl functional group.
